# EUROPEAN PATENT APPLICATION

(11) **EP 3 378 502 A1**
(43) Date of publication of application: **26.09.2018**
(21) Application number: 17162567.6
(22) Date of filing: 23.03.2017
(51) Int. Cl.: A61L 15/18, A61L 15/46

(54) **USE OF ZINC TREATED PRECIPITATED CALCIUM CARBONATE IN HYGIENIC PRODUCTS**

(71) Applicant: Omya International AG, 4665 Oftringen (CH)
(72) Inventor: OHR, Steffen, 6210 Sursee (CH); SCHRUL, Christopher, 4663 Aarburg (CH)
(74) Representative: Maiwald Patent- und Rechtsanwaltsgesellschaft mbH

(57) **Abstract**

The present invention relates to the use of zinc treated precipitated calcium carbonate (PCC), which is obtained by slaking calcium oxide with water to obtain a calcium hydroxide slurry, carbonating the calcium hydroxide slurry, and adding a Zn²⁺ ion provider before and/or during the carbonation, in hygienic products, to the hygienic products comprising said zinc treated precipitated calcium carbonate as well as to a process for the preparation of such hygienic products.

## Description

The present invention relates to the use of zinc treated precipitated calcium carbonate (PCC) in hygienic products, as well as to the hygienic products comprising this zinc treated precipitated calcium carbonate.

Hygienic products such as diapers, feminine hygiene products, incontinence products, etc. are valuable means for protecting the persons in need of such products from body liquids the release of which for various reasons cannot be controlled.

Modem disposable baby diapers and incontinence products have a layered construction, which allows the transfer and distribution of urine to an absorbent core structure where it is locked in. Basic layers may be an outer shell of breathable film or a nonwoven and film composite which prevents wetness and soil transfer, an inner absorbent layer of a mixture of air-laid paper and superabsorbent polymers for wetness, and a layer nearest the skin of nonwoven material with a distribution layer directly beneath which transfers wetness to the absorbent layer.

Thus, the use of such hygienic products makes life much easier for any one in need for it. There are, however, also disadvantages, such as skin irritation, commonly referred to as diaper dermatitis, and others.

Irritant diaper dermatitis develops when skin is exposed to prolonged wetness, increased skin pH caused by urine and faeces, and resulting breakdown of the stratum corneum, or outermost layer of the skin. This may be due to diarrhea, frequent stools, tight diapers, overexposure to ammonia, or allergic reactions. Thus, skin irritations may not only occur when diapers are used, but, generally with any hygienic product.

The most effective treatment is to discontinue use of the hygienic product, allowing the affected skin to air out. Thorough drying the skin before contacting it with the hygienic product is a good preventive measure, because it is the excess moisture, VT either from urine and faeces or from sweating or other body liquids, that sets the conditions for a dermatitis to occur. Various moisture-absorbing powders, such as talcum or starch, reduce moisture but may introduce other complications. Airborne powders of any sort can irritate lung tissue, and powders made from starchy plants (corn, arrowroot) provide food for fungi and are not recommended.

Another approach is to block moisture from reaching the skin, and commonly recommended remedies using this approach include oil-based protectants or barrier cream, various over-the-counter "diaper creams", petroleum jelly, dimethicone and other oils. Such sealants sometimes accomplish the opposite, if the skin is not thoroughly dry, in which case they serve to seal the moisture inside the skin rather than outside.

Also, zinc oxide-based ointments are quite effective, especially in prevention, because they have both a drying and an astringent effect on the skin, being mildly antiseptic without causing irritation.

Thus, there are a number of pharmaceutical zinc containing formulations for treating the skin before the hygienic product is applied.

This, however, has several disadvantages. For example, zinc ointments must be reapplied every time the hygienic is replaced and the skin is cleaned, excess ointment may contaminate clothes such as underwear, further ingredients in the ointments may cause irritations such as allergic reactions, etc.

Thus, there is a continuous need to reduce the risk of skin irritations caused by the use of hygienic products, at a minimum effort for the user and a maximum efficiency as regards the protection of the skin, ideally combined with other properties such as the control of odour.

Searching for a solution to these problems, it has now been found that certain zinc treated mineral material, especially zinc treated precipitated calcium carbonate, may advantageously be used in hygienic products and thus provide an effective means of preventing skin irritations directly by their incorporation into the hygienic product, and thus avoid any one of the above described disadvantages of the prior art.

Accordingly, the present invention relates to the use of zinc treated precipitated calcium carbonate (PCC) in hygienic products, wherein the zinc treated precipitated calcium carbonate is obtained by
- slaking calcium oxide with water to obtain a calcium hydroxide slurry,
- carbonating the calcium hydroxide slurry,
- adding a Zn²⁺ ion provider before and/or during the carbonation.

"Precipitated calcium carbonate" (PCC) in general is a synthesized material, obtained by precipitation following a reaction of carbon dioxide and calcium hydroxide (hydrated lime) in an aqueous environment or by precipitation of a calcium- and a carbonate source in water. Additionally, precipitated calcium carbonate can also be the product of introducing calcium- and carbonate salts, calcium chloride and sodium carbonate for example, in an aqueous environment. PCC may have a vateritic, calcitic or aragonitic crystalline form. PCCs are described, for example, in EP 2 447 213 A1, EP 2 524 898 A1, EP 2 371 766 A1, EP 2 840 065 A1, or WO 2013/142473 A1.

Subsequently, the calcium hydroxide slurry may be screened to remove any residual impurities and/or non-reactive unburnt lime, and then carbonated by reacting the calcium hydroxide with carbon dioxide or soluble carbonates.

According to the present invention, in a first step, calcium oxide may be slaked with water as usual according to methods well-known in the art.

The calcium oxide used according to the present invention may be any commercially available product, or may be obtained by calcining calcium compounds, especially calcium carbonate. Calcination is a thermal treatment process, especially applied to calcium carbonate containing materials in order to bring about a thermal decomposition resulting in the formation of calcium oxide and gaseous carbon dioxide.

Calcium carbonate comprising material which may be used for calcination may be any calcium carbonate comprising materials such as those selected from the group comprising precipitated calcium carbonates or natural calcium carbonate containing minerals such as marble, limestone and chalk.

Calcium carbonate decomposes at about 1000 °C to calcium oxide. The calcination step may be carried out under conditions and using equipment well-known to the person skilled in the art. Generally, calcination may be carried out in furnaces or reactors (sometimes referred to as kilns) of various designs including shaft furnaces, rotary kilns, multiple hearth furnaces, and fluidized bed reactors.

The end of the calcination reaction may be determined, e.g. by monitoring the density change, the residual carbonate content, e.g. by x-ray diffraction, or the slaking reactivity by common methods.

The slaking step, i.e. the combination of calcium oxide with water to obtain calcium hydroxide, may, in principle, be carried out according to known methods, as well.

The calcium oxide may be added to the water in a weight ratio of CaO : water of between 1 : 3 and 1 : 20, preferably between 1 : 5 and 1 : 12, most preferably between 1 : 7 and 1 : 10.

The progress of the reaction may be observed by measuring the conductivity of the reaction mixture, which initially quickly decreases and reaches an essentially constant level as soon as the reaction is completed. As well, it may be monitored by temperature and turbidity control.

The obtained calcium hydroxide slurry may be screened, e.g. on a 100 µm screen as appropriate, to remove any residual impurities and/or non-reactive unburnt lime.

For obtaining the zinc treated precipitated calcium carbonate used according to the invention the carbonation step is carried out in the presence of at least one Zn²⁺ ion provider.

The Zn²⁺ ion provider preferably is a water soluble compound, wherein, for the purpose of the present application, "water-soluble" compounds are defined as materials which, when 100 g of said material is mixed with 100 g deionised water and filtered on a filter having a 0.2 µm pore size at 20 °C to recover the liquid filtrate, provide more than 0.1 g of recovered solid material following evaporation at 95 to 100 °C of 100 g of said liquid filtrate at ambient pressure.

The Zn²⁺ ion provider may be selected from the group comprising zinc sulphate; zinc halides such as zinc chloride, zinc bromide, zinc iodide; zinc nitrate; zinc phosphates, e.g. Zn₃(PO₄)₂, ZnHPO₄; zinc carbonates, e.g. ZnCO₃, Zn(HCO₃)₂; zinc oxide; zinc hydroxide; hydrates and mixtures thereof. Especially preferred is the use of zinc sulphate heptahydrate (ZnSO₄ · 7 H₂O).

It is preferred that the Zn²⁺ ion provider is added in an amount of from 0.1 to 30 wt%, preferably of from 2 to 25 wt%, more preferably of from 5 to 20 wt%, most preferably of from 10 to 15 wt%, e.g. 11.5 wt% based on the dry weight of calcium oxide.

In a further preferred embodiment, the Zn²⁺ ion provider is added in combination with one or more Group I and/or Group II and/or Group III metal sulphates, which are preferably selected from the group comprising sodium sulphate, calcium sulphate, magnesium sulphate, aluminium sulphate; hydrates and mixtures thereof. Especially preferred is a combination with magnesium sulphate heptahydrate (MgSO₄ · 7 H₂O).

The one or more Group I and/or Group II and/or Group III metal sulphates advantageously are added in an amount of from 0.1 to 30 wt%, preferably of from 1 to 25 wt%, more preferably of from 2 to 15 wt%, most preferably of from 4 to 8 wt%, e.g. 6 wt% based on the dry weight of calcium oxide.

The Zn²⁺ ion provider and/or the Group I and/or Group II and/or Group III metal sulphates, independently from each other may be added before and/or during the carbonation.

It is, however, preferred that the Zn²⁺ ion provider and/or the Group I and/or Group II and/or Group III metal sulphates are added after the slaking step.

It may be especially advantageous, if the Zn²⁺ ion provider, especially zinc sulphate, is added during the carbonation step, and the Group I and/or Group II and/or Group III metal sulphate, especially magnesium sulphate, is added before the carbonation step.

Furthermore, in some embodiments, one or more acids may be added before and/or during the carbonation step, e.g. sulphuric acid.

The carbonation of the calcium hydroxide suspension may be carried out by feeding pure gaseous carbon dioxide or technical gases containing at least 10 vol.% of carbon dioxide into the alkaline calcium chloride solution. In this respect, it is possible to use any technical flue gases, provided that they do not contain any components causing undesired side reaction, or introducing impurities in the process of the present invention.

The carbonation is carried out by means and under conditions and equipment well-known by the person skilled in the art.

In an especially preferred embodiment, however, the carbonation is conducted at a carbonation gas flow rate of below 30 litres per minute, preferably at a rate of from 1 to 30, more preferably of from 10 to 20, most preferably about 20 litres per minute, and/or preferably at a temperature of from 10 to 70 °C, more preferably 15 to 50 °C, and most preferably 20 to 30 °C during precipitation.

The progress of the carbonation reaction can be readily observed by measuring the conductivity density, turbidity and/or pH.

In this respect, the pH of the calcium hydroxide suspension before addition of carbon dioxide will be more than 10 and will constantly decrease until a pH of below 8 is reached. At this point the reaction can be stopped.

Conductivity slowly decreases during the carbonation reaction and rapidly decreases to low levels, when the precipitation is completed.

The temperature in the carbonation tank is observed to rise up to between 40 and 80 °C, preferably up to between 50 and 60 °C, most preferably up to between 56 and 57 °C.

The final slurry product consists of a slurry of zinc treated precipitated calcium carbonate and may have a solids content of 1 to 30 wt%, preferably 5 to 25 wt%, more preferably 10 to 20 wt%, most preferably 15 to 18 wt%.

In a preferred embodiment, the zinc treated precipitated calcium carbonate is in the form of agglomerates and/or aggregates.

Agglomerates in the meaning of the present invention are accumulations of weakly bound particles or aggregates or mixtures thereof, the resulting surface of which is comparable to the sum of the surfaces of the respective components. Agglomerates are held together by weak forces, e.g. van-der-Waals forces or ordinary physical catching. Agglomerates are also designated as secondary particles, the original particles as primary particles (cf. e.g. DIN SPEC 1121 (DIN ISO/TS 27687), Deutsches Institut für Normung e.V.).

Aggregates in the meaning of the present invention are strongly bound or fused particles, the resulting surface of which may be significantly smaller than the sum of the calculated surfaces of the respective components. Aggregates are held together by strong forces, such as covalent forces or forces based on sintering or complex physical catching. Also aggregates are designated as secondary particles. (cf. e.g. DIN SPEC 1121 (DIN ISO/TS 27687), Deutsches Institut für Normung e.V.).

The slurry of zinc treated precipitated calcium carbonate may be concentrated by means of pressurized filters, centrifuges, vacuum filtration, thermal evaporation, optionally in the presence of cationic and/or anionic dispersants, preferably until a solids content of from 15 to 60 wt%, more preferably of from 20 to 55 wt%, especially preferably of from 25 to 50 wt%, most preferably of from 30 to 45 wt%, e.g. 35 to 40 wt% is obtained, or until dryness.

The amount of optional dispersant added is controlled so that the PCC is just coated, this quantity corresponding to that added prior to a slurry viscosity increase. Dispersants, which may be advantageously used are those well-known in the art, and preferably are selected from the group comprising sodium, potassium, calcium, magnesium, lithium, strontium, primary amine, secondary amine, tertiary amine and/or ammonium salts, whereby the amine salts are linear or cyclic, of at least partly neutralized homopolymers or copolymers of (meth)acrylic acid, maleic acid, fumaric acid, itaconic acid and derivatives of these acids, preferably esters or amides such as methyl methacrylate, methyl acrylate, acrylamide, sodium hydrogen phosphate or polyphosphates such as alkalipolyphosphates, carboxymethylcellulose, steric dispersants, comb polymers, and/or mixtures thereof, preferably sodium polyacrylate having a molecular weight M_{w} of from 4 000 to 10 000 g/mol, preferably from 4 000 to 8 000 g/mol and most preferably of about 6 000 g/mol.

Generally, dispersants in an amount of from 0.001 to 10 wt%, preferably from 0.1 to 7 wt%, more preferably from 0.5 to 6 wt%, especially from 1 to 5 wt%, e.g. from 3 to 4 wt% may be added based on the dry weight of calcium carbonate.

For example, approximately 5 to 10 wt% of a 40 wt% solution of a sodium salt of polyacrylic acid relative to dry calcium carbonate may be added to the slurry or filter cake containing the zinc treated precipitated calcium carbonate of the invention, corresponding to approximately 2 to 4 wt% dry polyacrylic acid salt on dry calcium carbonate.

Before concentrating the slurry, it may be screened, e.g. on a 45 µm screen, in order to remove coarse residual impurities and/or non-reactive unburnt lime, when the Brookfield viscosity of the material exiting from the carbonation tank is sufficiently low, namely less than 100 mPa·s at 100 rpm.

The concentrated slurry may be washed and, subsequently, re-dispersed, optionally in the presence of one or more dispersants as mentioned above, such as alkali salts of polyacrylic acid, carboxy methyl cellulose, mixtures of carboxy methyl cellulose with sodium citrate, etc.

The precipitated calcium carbonate may have aragonitic, calcitic, or vateritic crystal structure, or mixtures thereof, wherein the crystal structure and morphology of the precipitated calcium carbonate can optionally be controlled, e.g. by addition of seed crystals or other structure modifying chemicals.

Aggregates / agglomerates of zinc treated precipitated calcium carbonate used according to the present invention may have a volume median aggregate / agglomerate diameter *d*₅₀(vol) of from 0.1 to 10 µm, more preferably 0.25 to 8 µm, most preferably 0.5 to 7 µm, especially 1 to 6 µm, e.g. 4 to 5.5 µm.

The primary acicular particle size preferably is in the nanometer range, and may be from 1 to 100 nm, preferably from 10 to 80 nm, more preferably from 20 to 50 nm.

The BET specific surface area of the aggregates / agglomerates of zinc treated precipitated calcium carbonate according to the present invention may be from 1 to 100 m²/g, preferably from 20 to 85 m²/g, more preferably from 40 to 80 m²/g, especially from 60 to 75 m²/g, e.g. 70 m²/g, measured using nitrogen and the BET method according to ISO 9277.

Methods for the preparation of zinc treated precipitated calcium carbonate being especially useful for the use according to the present invention are e.g. described in European patent applications EP 1 712 523 A1 and EP 1 712 597 A1, where they are used as pigments in paper coating formulations to manufacture coated high-quality matt or multipurpose papers, in particular for inkjet applications, providing print qualities essentially identical with or close to high resolution commercial papers. The use of such pigments in hygienic products, however, is not mentioned.

For the use in hygienic products according to the present invention, the zinc treated precipitated calcium carbonate may be used in the form of a slurry, a filter cake or dry as described above, or in the form of a formulation suitable for such applications, especially an aqueous formulation, preferably selected from the group comprising coating formulations, spray coating formulations, lotions, and printing ink formulations.

For preparing a corresponding formulation, the zinc treated precipitated calcium carbonate advantageously is provided dry, in the form of a filter cake, or in the form of an aqueous slurry.

The final formulation may have a solids content of from 15 to 70 wt%, preferably 20 to 60 wt%, more preferably 30 to 40 wt%, e.g. 32 wt%, and/or a viscosity of from 50 to 3000 mPa·s, preferably from 100 to 2000 mPa·s, more preferably from 200 to 1000 mPa·s, especially preferably from 300 to 700 mPa·s, most preferably from 400 to 600 mPa·s, e.g. 460 mPa·s.

A corresponding formulation may comprise further components, preferably selected from the group comprising dispersing agents, binding agents, biocides, defoamers, oils, pigments, coalescing agents, wetting agents, neutralizing agents, emulsifiers, solvents, colorants, and mixtures thereof.

Accordingly, the zinc treated precipitated calcium carbonate may be applied to hygienic products in the form of a slurry, dry, or in the form of a formulation.

Such products may be selected from ab/adsorbent products such as diapers, training panties, swim pants, feminine hygiene products such as pads, panty liners, sanitary napkins, incontinence products; deodorant formulations; nonwoven products such as wipes.

In an especially preferred embodiment the zinc treated precipitated calcium carbonate in the form of a slurry, dry or in the form of a formulation is applied to an ab/adsorbent product comprising one or several layers selected from the group comprising a top sheet layer, one or more acquisition/distribution layer(s) (ADLs), a tissue wrap layer, an ab/adsorbent core and a back sheet layer.

Preferably, the zinc treated precipitated calcium carbonate in the form of a slurry, dry or in the form of a formulation may be applied to one or several of the layers of the ab/adsorbent product, selected from the group comprising a top sheet layer, one or more acquisition/distribution layer(s) (ADLs), a tissue wrap layer, an ab/adsorbent core and a back sheet layer.

The application of the zinc treated precipitated calcium carbonate may be carried out by any well-known means being suitable therefor such as spray coating, roller coating, blade coating, curtain coating, etc.

The amount of the zinc treated precipitated calcium carbonate will depend on the respective formulation. It may be preferred that the zinc treated precipitated calcium carbonate is applied to provide a dry coating weight of 0.05 to 15 mg/cm², preferably 0.1 to 10 mg/cm², more preferably 0.2 to 5 mg/cm², most preferably 0.5 to 4 mg/cm², e.g. 1 to 2 mg/cm².

In hygienic products, it is especially preferred to use the zinc treated precipitated calcium carbonate in the form of a spray coating formulation.

A corresponding spray coating formulation preferably is an aqueous spray coating formulation and may comprise binders, such as carboxymethyl cellulose binders available under the tradename Finnfix^{®} CMC 5 from CPKelco.

In spray coating formulations, such binders preferably are used in an amount of from 1 to 30 wt%, preferably from 5 to 25 wt%, more preferably from 7.5 to 20 wt%, especially from 10 to 15 wt%, e.g. 12 wt% based on the weight of dry calcium carbonate.

It may comprise further components, as mentioned above, especially defoaming agents in appropriate amounts.

An especially suitable spray coating formulation has a solids content of from 25 to 35 wt%, e.g. 31 to 32 wt%, and/or a Brookfield viscosity (100 rpm, Spindle 4) of from 300 to 500 mPa·s, e.g. 300 to 460 mPa·s.

It may be applied by any equipment suitable for spray coating. Generally different spray nozzles geometries can be used e.g. full cone nozzle, hollow cone nozzle, fan nozzle, nozzle providing a full beam or systems working with air atomization (e.g. available from BETE Deutschland GmbH, Lechler GmbH, DIVA-Sprühtechnik GmbH), preferably nozzles using the dual beam technology (available e.g. from FMP TECHNOLOGY GMBH).

Accordingly, the hygienic products comprising zinc treated precipitated calcium carbonate as described above are a further aspect of the present invention.

Such hygienic products preferably are selected from the group comprising ab/adsorbent products such as diapers, training panties, swim pants, feminine hygiene products such as pads, panty liners, sanitary napkins, incontinence products; deodorant formulations; nonwoven products such as wipes.

Hygienic products such as ab/adsorbent products, e.g. diapers may comprise one or several layers selected from the group comprising a top sheet layer, one or more acquisition/distribution layer(s) (ADLs), a tissue wrap layer, an ab/adsorbent core and a back sheet layer, wherein the zinc treated precipitated calcium carbonate as defined above may be applied to at least one of said layers.

A further aspect of the present invention accordingly is a process for the preparation of corresponding hygienic products, wherein the zinc treated precipitated calcium carbonate may be applied to the hygienic products in the form of a slurry, dry, or in the form of a formulation.

In an especially preferred embodiment, the zinc treated precipitated calcium carbonate is applied by spray coating or printing, e.g. flexographic printing, on one or several of the above-mentioned layers of a diaper or any other ad/absorbent product.

The following figures, examples and tests will illustrate the present invention, but are not intended to limit the invention in any way.

### Figures

- Figure 1: illustrates the crystalline structure of aggregates / agglomerates of zinc treated precipitated calcium carbonate determined by an SEM image.
- Figure 2: illustrates a process for spray coating a substrate with zinc treated precipitated calcium carbonate by the dual beam spray technology.
- Figure 3: illustrates the structure of a typical baby diaper.
- Figure 4: illustrates a flexo print unit.
- Figures 5a to 5c: show different magnifications of SEM images of a diaper PP (polypropylene) topcoat having a connection stripe joining the PP fibres (Fig. 5a and 5b), and of single PP fibres (Fig. 5c).
- Figures 6a and 6b: show different magnifications of SEM images of a diaper PP topcoat having a connection stripe joining the PP fibres and zinc treated precipitated calcium carbonate bonded on the surface according to V14.
- Figure 7: shows an SEM image of a group of PP fibres of a diaper PP topcoat having a wide distribution of zinc treated precipitated calcium carbonate bonded to the surface according to V13.
- Figures 8a and 8b: show SEM images of a diaper PP topcoat having a connection stripe joining the PP fibres and zinc treated precipitated calcium carbonate bonded on the surface (Fig. 8a) and a group of PP fibres having a wide distribution of zinc treated precipitated calcium carbonate bonded to the surface (Fig. 8b) according to V17.

### EXAMPLES

### 1. Measurement methods

### BET specific surface area (SSA)

The BET specific surface area was measured via the BET process according to ISO 9277 using nitrogen, following conditioning of the sample by heating at 250 °C for a period of 30 minutes. Prior to such measurements, the sample was filtered, rinsed and dried at 110 °C in an oven for at least 12 hours.

**Particle / Aggregate / Agglomerate size distribution (volume % particles** / **aggregates / agglomerates with a diameter < *X*), *d*₅₀ value (volume median particle / aggregate / agglomerate diameter) and *d*₉₈ value of a particulate material:**
The volume median particle or aggregate or agglomerate diameter and particle or aggregate or agglomerate diameter volume distribution of a particulate calcium carbonate-containing material was determined via laser diffraction, i.e. the particle or aggregate or agglomerate size is determined by measuring the intensity of light scattered as a laser beam passes through a dispersed particulate sample. The measurement was made with a HELOS particle-size-analyzer of Sympatec, Germany. The samples were prepared as follows: In case that the particulate calcium carbonate containing material was a powder, the powder was mixed with demineralized water to form a homogenous slurry having a solids content in the range of 20 to 25 wt.%. In case that the particulate calcium carbonate-containing material already was in the form of a slurry, it was brought to a solids content in the range of 20 to 25 wt.% by use of demineralized water, if necessary. Before starting the measurement it was ensured that the slurry to be measured did not contain any sediments. Alternatively, the measurement can be made with a Mastersizer 2000 or a Mastersizer 3000 of Malvern Instruments Ltd. (operating instrument software version 1.04).

### Solids content of an aqueous suspension

The suspension solids content (also known as "dry weight") was determined using a Moisture Analyser MJ33 from the company Mettler-Toledo, Switzerland, with the following settings: drying temperature of 160 °C, automatic switch off, if the mass does not change more than 1 mg over a period of 30 seconds, standard drying of 5 to 20 g of suspension.

### PH of an aqueous suspension

The pH of a suspension or solution was measured at 25 °C using a Mettler Toledo Seven Easy pH meter and a Mettler Toledo InLab® Expert Pro pH electrode. A three-point calibration (according to the segment method) of the instrument was first made using commercially available buffer solutions having pH values of 4, 7 and 10 at 20 °C (from Sigma-Aldrich Corp., USA). The reported pH values are the endpoint values detected by the instrument (the endpoint was when the measured signal differed by less than 0.1 mV from the average over the last 6 seconds).

### Brookfield Viscosity

For the purpose of the present invention, the term "viscosity" or "Brookfield viscosity" refers to Brookfield viscosity. The Brookfield viscosity is for this purpose measured by a Brookfield DV-III Ultra viscometer at 24°C ± 3°C at 100 rpm using an appropriate spindle of the Brookfield RV-spindle set and is specified in mPa·s. Once the spindle has been inserted into the sample, the measurement is started with a constant rotating speed of 100 rpm. The reported Brookfield viscosity values are the values displayed 60 seconds after the start of the measurement. Based on his technical knowledge, the skilled person will select a spindle from the Brookfield RV-spindle set which is suitable for the viscosity range to be measured. For example, for a viscosity range between 200 and 800 mPa·s spindle number 3 may be used, for a viscosity range between 400 and 1600 mPa·s spindle number 4 may be used, for a viscosity range between 800 and 3200 mPa·s spindle number 5 may be used, for a viscosity range between 1000 and 2000000 mPa·s spindle number 6 may be used, and for a viscosity range between 4000 and 8000000 mPa·s spindle number 7 may be used.

### SEM images

Scanning electron micrographs (SEM) were carried out by diluting 150 µl sample slurry with 20 ml deionized water, filtering through a 0.8 µm filter, and air drying the filter residue. A sample carrier provided with carbon based, electrically conductive, double sided adhesive discs is pressed onto the filter, and, subsequently, sputtered with gold (8 nm) and evaluated in the SEM at various enlargements.

### 2. Material

### 2.1. Zinc treated precipitated calcium carbonate (Zn-PCC)

150 kg of quicklime were added to 1300 litres of tap water having a temperature of 40 °C in a stirred reactor and slaked for 25 minutes under continuous stirring. The resulting slurry of calcium hydroxide ("milk of lime") at 12.8 wt% solids was then screened on a 100 µm screen.

The calcium carbonate precipitation was carried out in a 1000 litre baffled cylindrical stainless steel reactor equipped with a gassing agitator having a gas dispersion unit, a stainless steel carbonation tube to direct a carbon dioxide/air gas stream to the impeller, and probes for monitoring the pH and conductivity of the suspension.

700 litres of the screened calcium hydroxide slurry were added to the carbonating reactor and the temperature of the reaction mixture was adjusted to the desired starting temperature of 20 °C.

Before starting the carbonation reaction, 30 kg of 10 wt% aqueous solution of magnesium sulphate (MgSO₄ · 7H₂O) was added to the milk of lime.

The agitator was then adjusted to 1480 rpm, and the slurry was carbonated by passing a gas mixture of 26 vol.% carbon dioxide in air at 118 Nm³/h, corresponding to 19.7 litres per minute at standard temperature (0 °C) and pressure (1000 mbar) per kilogram of calcium hydroxide, through the slurry.

During carbonation, 100 kg of 10 wt% aqueous solution of zinc sulphate (ZnO₄ · 7 H₂O) were added continuously over the total carbonation time to the reaction mixture.

Completion of carbonation was reached after 1 hour 50 minutes reaction time and indicated by a drop in conductivity to a minimum accompanied by a drop in pH to a constant value below 8.

During carbonation the slurry temperature was allowed to rise resulting in a final slurry temperature of 58 °C due to the heat generated during the exothermic reaction.

The slurry was then screened on a 45 µm screen before being fed to a dewatering centrifuge (operating at 4440 rpm) at a rate of 350 l/h providing a filter cake having a solids content of 42 wt%. This filter cake was collected and then redispersed with 2.5 wt% (active/dry) of a 100 % sodium neutralized polyacrylate dispersing agent (Mw ~ 12000 g/mol; polydispersity index ~ 3) in a mixing unit and the concentrated product was recovered as an aqueous slurry of the pigment.

The product of the carbonation and concentration step as stated above was an aqueous suspension of 38 wt% solids content of ultrafine primary calcium carbonate particles bound together to form aggregates / agglomerates.

Further properties of the obtained product are described in table 1 below.

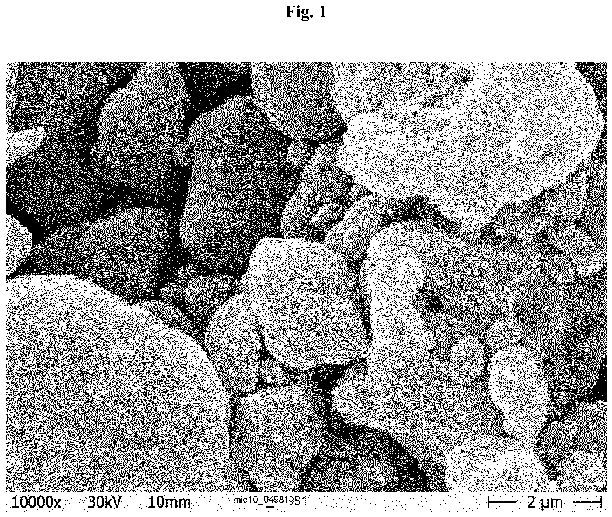

The crystalline structure of the product was determined by SEM pictures and is exemplified by Figure 1.

### 2.2. Spray Coating Formulations

From the above described zinc treated precipitated calcium carbonate having a solids contents of 38 wt%, a spray coating formulation was prepared by adding a carboxymethyl cellulose binder available under the tradename Finnfix^{®} CMC 5 from CP Kelco in a high shear mixer (Disperlux Pendraulik lab dissolver) for 15 minutes without temperature regulation, and 3000 rpm with a dispersion disc with a diameter of 60 mm, which, before its addition to the zinc treated precipitated calcium carbonate suspension, has been stirred with a paddle stirrer at a temperature of 90 °C in tap water at a solids content of 15 to 20 wt% for 20 to 30 min.

The composition and characteristics of the resulting coating formulation are summarized below:

**Table 1:**

| | **Formulation** |
|---|---|
| **Composition** | |
| Zn treated PCC [dry wt%] | 25.5 |
| Finnfix^{®} CMC 5 [dry wt%] | 4.7 |
| Dispersing agent [dry wt%] | 1.3 |
| Water [wt%] | 68.5 |

| **Particle Size Distribution of the Zn-PCC in the formulation** | |
|---|---|
| ***d***₅₀(vol) [µm] | 5.0 |

| **BET Specific Surface Area of the Zn**-**PCC in the formulation** | |
|---|---|
| [m²/g] | 70 |

| **Fluid characteristics of the formulation** | |
|---|---|
| Solids Content [wt%] | 31.6 |
| pH | 9.6 |
| Brookfield Viscosity 100 rpm, Spindle 4 [mPa·s] | 460 |

### 3. Pre-Trials

As it is essential to provide the hygienic product, such as a diaper, in an uncomplicated and flexible way with the zinc treated precipitated calcium carbonate containing coating formulation, the sprayability of the formulations was tested.

For this purpose, first, the dual beam technology was applied in order to generally test the sprayability of the coating formulations, which showed excellent atomization behaviour. Based on these results, the formulation was applied to diapers.

### 3.1. Sprayability

For testing the sprayability of the coating formulations, the following parameters were set to be met for being suitable to be applied on hygienic products such as diapers:

**Table 2:**

| | |
|---|---|
| Coat weight (wet) | 200 to 1000 mg |
| Coat weight (dry) | 60 to 310 mg |
| Web speed | 330 m/min |
| Coating area length on substrate | 185 ± 15 mm |
| Coating area width on substrate | 45 ± 5 mm |
| Pulsation / spray burst | 760 n/min |
| Nozzle opening time | 34 ± 3 ms |

For the trial, standard equipment applying the dual beam spray-technology was used, wherein purpose-built nozzles as described further below were used to realize the coat weight and coating area which was defined as mentioned above.

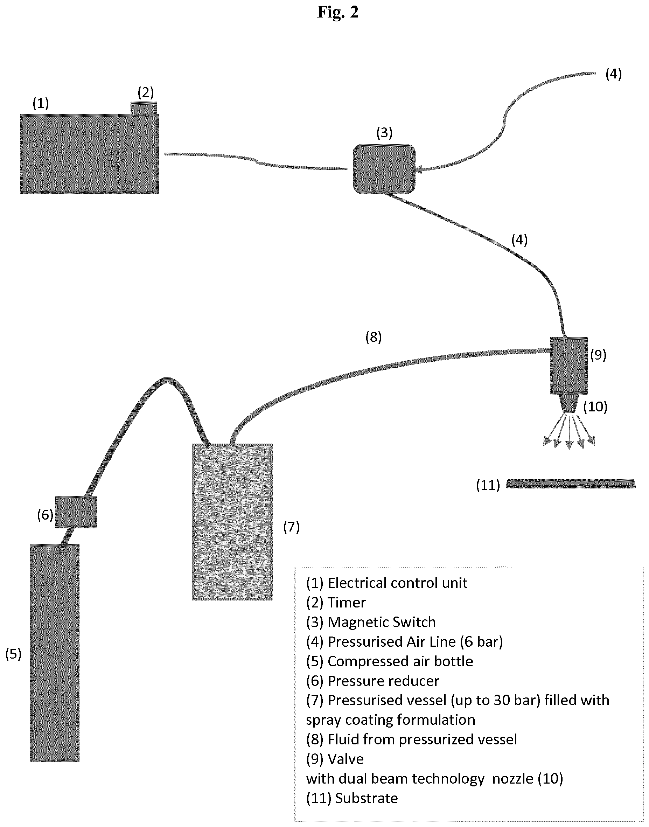

The formulations can be sprayed on any defined substrate using state of the art spray technology. As illustrated in Figure 2, the formulation was transferred into a stainless steel vessel with a capacity of 1000 ml. The vessel can be pressurized with compressed air to a maximum pressure of 30 bar. From the vessel, the fluid was transported to a standard spraying valve (suppliers are for example: JERKEL or NORDSON or WALTHER). In this example, a WALTHER Pilot valve was used. To control the opening time of the valve, an electrical control unit was used giving a signal to the valve and allowing the valve to open for a defined period of time. Manual triggering was realized by an electrical control unit (1) (24V, max 4.2 amps), linked with a timer (2) (Panasonic LT4H). The timer was set to 94 ms. The control unit gives electrical impulse to a magnet switch (3), which opens the 6 mm pipe connected to the compressed air line (4) with 6 bar pressure. The magnetic switch was connected (4) to the valve (9), which by triggering the control unit, opened the valve for 34 ms and released the fluid (8) coming from the pressurized vessel (7). The vessel was pressurised by a bottle with compressed air at 250 bar (5) connected to a pressure reducer (6) to allow a reduction of the pressure vessel below 30 bar. At the valve opening, a nozzle (10) delivered by FMP TECHNOLOGY GMBH was used having two holes drilled in an angle (20 °) to each other, allowing the mass flow defined by the pressure of the vessel and the defined opening time of the valve to be separated in two beams, the two beams collide outside of the nozzle and atomize or spread the fluid. The advantage of this nozzle is, that no additional pressurized air is needed to atomize or spread the fluid. The nonwoven substrate (11) (a low bonded, hydrophilic polypropylene nonwoven having a weight of 14 g/m² was coated by fixing it on a substrate carrier and placing it on a conveyor belt. The conveyor belt was set to a speed of 100 m/min. The substrate on the substrate carrier was subsequently transported and passed the valve/nozzle. To coat the substrate on the substrate carrier, the opening signal from the electrical control unit to the valve was manually triggered by a rocker switch when the substrate passed the valve underneath.

As regards the nozzles, two nozzle sizes were tested, 500 µm and 650 µm, and for both nozzles, the atomization was found to be excellent.

The mass output, i.e. the wet coat weight per piece, was controlled by varying the pressure of the vessel mass outputs at the given parameters such as nozzle opening time given above.

The spray coating of the coating formulation was carried out using two nozzle sizes and different pressure settings of the vessel. After the trials, the dry coat weight was determined by drying the coated material stored in the lab under ambient temperature (22 °C) and humidity (ca. 45 %) for 20 h and determining its weight. The difference between the dried coated nonwoven sheet and the dried uncoated nonwoven sheet corresponds to the dry coat weight.

**Table 3:**

| **Trial** | **Vessel Pressure [bar]** | **Nozzle Diameter [µm]** | **Coat Weight (dry) [mg]** |
|---|---|---|---|
| V1 | 24 | 650 | 404 |
| V2 | 19 | 650 | 274 |
| V3 | 15 | 650 | 204 |
| V4 | 15 | 500 | 134 |
| V5 | 12 | 500 | 124 |
| V6 | 9 | 500 | 74 |

Accordingly, the target dry coat weight could be realized within the trial (V2 to V6).

Furthermore, considering the solids content of coating formulation 1 of 31.6 wt%, the achieved dry coat weights of V4, V5 and V6 displayed the calculated mass output. This finding indicates that almost all solids content remains on the surface of the nonwoven and is not penetrating through its pores.

### 3.2. Zinc retention trials

For evaluating the amount of zinc remaining on the spray coated non-woven the same trials were carried out as described above with the following exceptions:
The coating formulation was applied and dry coat weights from 74 mg to 404 mg per piece were achieved. The remaining solids residue after ignition and the corresponding zinc content determined by the ICP MS method is displayed in the table below.

**Table 4**

| **Trial** | **Vessel Pressure [bar]** | **Nozzle Diameter [µm]** | **Coat Weight (dry) [mg]** | **Top Sheet Weight Residue on Ignition (570 °C) [g]** | **Zn Content [%]** |
|---|---|---|---|---|---|
| V1 | 24 | 650 | 404 | 0.3391 | 0.37 |
| V2 | 19 | 650 | 274 | 0.2144 | 0.35 |
| V3 | 15 | 650 | 204 | 0.1574 | 0.26 |
| V4 | 15 | 500 | 134 | 0.1147 | 0.25 |
| V5 | 12 | 500 | 124 | 0.1023 | 0.22 |
| V6 | 9 | 500 | 74 | 0.0699 | 0.17 |

### 3.3. Determination of of the antimicrobial and antifungal activity of zinc treated precipitated calcium carbonate

The microbial contamination (bacterial and fungi growth) of three different zinc containing powder samples was determined by the plate count method (spread count) according to the following procedure:

### 3.3.1. Materials and Methods

### Zinc containing powders

The powders used were:
- ZnCO₃ (from Sigma-Aldrich)
- ZnO (from Sigma-Aldrich)
- Zn treated PCC (as described above)

### Disruption buffer (DB) preparation

This buffer was used to detach the bacterial cells from the zinc containing powder particles.

1.12 g of tris(hydroxymethyl)aminomethane (Tris) was dissolved in 800 ml of a 0.9 % (w/v) saline solution. The pH was adjusted to 8 with HCl and made up to 1 litre with 0.9 % (w/v) saline solution. The solution was then passed through a 0.2 µm pore filter or autoclaved (121 °C, 15 minutes) and aliquoted into 50 ml sterile tubes. The aliquots were stored at room temperature (21 °C).

### Sample preparation

1 g of the respective powder and 9 ml sterile disruption buffer (DB) were weighed into a sterile 50ml test tube (Greiner) under aseptic conditions using an autoclaved disposable spatula (VWR) in order to obtain suspensions of the zinc containing powder samples. To detach the microorganisms from the zinc containing powders samples, the suspensions were shaken on a vortex for 60 sec. at 2500 rpm before being put on a shaker for 30 minutes at motor setting 1400 (at room temperature, i.e. 21 °C).

### Planting procedure

The sample to be analyzed was appropriately diluted and a defined volume no greater than 0.1 ml was spread over the surface of an agar plate using a sterile glass / plastic spreader. The agar plates were then incubated upside down (condensed water) for a defined period of time at a defined temperature.

### a) Bacteria (aerobe total viable count)

The following parameters are specifically defined:
Diluent: Phosphate buffered saline (PBS) (from Fluka)
Agar: Tryptic Soy Agar plates (TSA) (ready made from Biomérieux)

The sample to be analyzed was diluted 1:10 in PBS and 100 µl of the dilution was spread onto the TSA plate which was incubated for 48 hours at 30 °C.

### b) Fungi

The following parameters are specifically defined:
Diluent: PBS (Phosphate buffered saline) (from Fluka)
Agar: Sabouraud-Glucose (4%)-Agar plate (with chloramphenicol) (from BIOTEST/Heipha)

The sample to be analyzed was diluted 1:10 in PBS and 100 µl of the dilution were spread onto the plate which was incubated for 7 days at 25 °C.

### Evaluation

The plates were evaluated by counting the grown colonies, thereby taking into account that only plates containing between 30 - 300 colonies have a high statistical significance.

The results are given in "cfu/ml" (colony-forming unit per ml sample) or "cfu/g" (colony-forming unit per g sample).

### 3.3.2. Results and Discussion

### Total viable count

**Table 5: Determination of aerobe bacterial and fungal growth.**

| | TVC [cfu/g] | |
|---|---|---|
| Sample | Aerobe¹⁾ | Fungi²⁾ |
| ZnCaCO₃ | < 100 | < 10 |
| ZnO | < 100 | < 10 |
| Zn-PCC | < 100 | < 10 |

| | | |
|---|---|---|
| ¹⁾ TSA: Tryptic Soy Agar for the determination of bacterial growth. ²⁾ SDC for the determination of fungal growth. 1ml was plated to lower the detection limit. | | |

As can be taken from table 5, no bacterial (detection limit 100 cfu/g) and no fungal (detection limit 10 cfu/g) contamination were found in the three samples.

This means that all of these samples show antimicrobial and antifungal activity, wherein the content of Zn in the Zn-PCC sample was the lowest indicating an increase of microbial effectivity by the use of Zn-PCC.

### 4. Application of zinc treated precipitated calcium carbonate in baby diapers

In the following trials, a spray coating formulation containing zinc treated precipitated calcium carbonate (PCC) was applied on baby diapers under industrial conditions, focussing on:
1. Sprayability with spray equipment installed in the machine at full speed
2. Impact of the spray coating on relevant diaper properties
3. Product stability and storage stability with respect to microbiological contamination.

The results of the determination of relevant diaper properties like rewet, absorption time, pH and microbiological investigations showed very good results.

### 4.1. Spray Coating Formulation

From the above described zinc treated precipitated calcium carbonate having a solids contents of 38 wt%, a spray coating formulation was prepared by adding a carboxymethyl cellulose binder available under the tradename Finnfix^{®} CMC 5 from CP Kelco in a high shear mixer (Disperlux Pendraulik lab dissolver) for 15 minutes without temperature regulation, and 3000 rpm with a dispersion disc with a diameter of 60 mm, which, before its addition to the zinc treated precipitated calcium carbonate suspension, has been stirred with a paddle stirrer at a temperature of 90 °C in tap water at a solids content of 15 to 20 wt% for 20 to 30 min.

The composition and characteristics of the resulting coating formulation are summarized below:

**Table 6:**

| | **Formulation** |
|---|---|
| **Composition** | |
| Zn treated PCC [dry wt%] | 27.4 |
| Finnfix^{®} CMC 5 [dry wt%] | 4.1 |
| Water [wt%] | 68.5 |

| **Fluid characteristics of the formulation** | |
|---|---|
| Solids Content [wt%] | 31.5 |
| pH | 10 |
| Brookfield Viscosity 100 rpm, Spindle 4 [mPa·s] | 600 |
| Temperature | 19 °C |

### 4.2. Spray Coating Procedure

The following parameters were set to be met for being suitable to be applied on diapers:

**Table 7:**

| | |
|---|---|
| Coat weight (wet) | 40 to 300 mg |
| Coat weight (dry) | 12.6 to 94.5 mg |
| Web speed | 330 m/min |
| Produced diapers | 760 n/min |
| Coating length on top sheet | 200 ± 15 mm |
| Coating width on top sheet | 45 ± 5 mm |
| Pulsation / spray burst | 760 n/min |
| Nozzle opening time | 34 ± 3 ms |

The trial was realized on a conventional industrial diaper line.

The spray coating equipment (valve carrier, valve, nozzle and connectors) was provided by FMP TECHNOLOGY GMBH as described above (cf. also Fig. 2).

Two nozzle sizes were chosen for the trial to transfer an adequate fluid amount onto the top sheet: 200 µm and 500 µm.

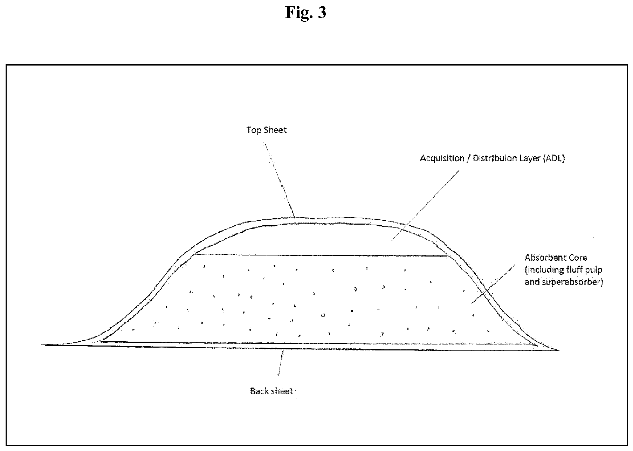

The diaper to be coated was a standard baby diaper maxi size 4 as illustrated in Fig. 3. The goal was to apply the spray coating formulation on the polypropylene top sheet.

The spray coating equipment was positioned in a section of the diaper line where the top sheet was already merged with the ADL (acquisition/distribution layer), absorbent core (fluff pulp and super-absorber layer) and the back sheet layer to allow the coating application onto the top sheet. Trial points V7 to V10 were run in this setup. During the trial, the application was slightly changed and adjusted to another position where the backside of the nonwoven top sheet could be coated. Trial point V11 was conducted in this setup. A dryer was not installed due to the limited space within the machine.

**Table 8**

| **Trial** | **Coating side on Top Sheet** | **Modus** | **Vessel Pressure [bar]** | **Nozzle Diameter [µm]** | **Coat Weight (wet) [mg]** |
|---|---|---|---|---|---|
| V7 | Top | Pulsing | 20 | 500 | 500 |
| V8 | Top | Pulsing | 10 | 500 | 250 |
| V9 | Top | Continuous | 9 | 200 | 113 |
| V10 | Top | Pulsing | 9 | 200 | 43 |
| V11 | Bottom | Pulsing | 9 | 200 | 43 |

### 4.3. Tests and Results

Important properties of the diaper were evaluated such as rewet, absorption time and pH. These methods are common in the industry and allow a performance evaluation with respect to the final application on babies. Furthermore, microbiological examinations have been carried out.

### 4.3.1. Rewet Test

The rewet test is designed to show the intrinsic ability of the absorbent core of a diaper to prevent fluids from resurfacing.

For evaluating the rewet properties of the treated diaper, 3 x 70 ml of a urine substitute (0.9 wt% NaCl solution) were poured with a delay of 20 min. after each addition onto each of the samples with a funnel. The micturition point is situated 2.5 cm ahead of the middle of the absorbent core. Between the beginning of the respective liquid application and the rewet measurement there was a delay of 20 min. in order to allow for the distribution and absorption of the liquid in the diaper sample. The surface moisture is determined quantitatively with a stack of filter papers of a known weight after 15 s under a weight of 4 kg. After having determined the final weight of the soaked filter papers, the weight of the liquid uptake could be calculated. The median values including standard deviation were indicated. A decreasing rewet value corresponds to an increasing surface dryness / skin friendliness as well as wearing comfort of the product.

As can be taken from table 9, the results of the rewet test indicate that a spray coated diaper shows an slightly improved performance compared to the reference diaper without coating. This means, that the ability of a diaper to hold the fluid and prevent it from resurfacing is unaffected. The target value was < 0.5 g.

**Table 9**

| | Rewet after production [g] | Rewet after drying [g] |
|---|---|---|
| Reference (untreated diaper) | 0.28 | 0.28 |
| V8 | 0.18 | 0.12 |
| V10 | 0.19 | 0.21 |

In this respect, "Rewet after production" means, a diaper sample was taken directly after the production process (ejection opening at the end of the machine), where the sample still had some residue moisture due to the coating. "Rewet after drying" means, that the diaper sample from the production trial was dried under ambient conditions until there was no residue moisture on the top sheet and was tested afterwards.

### 4.3.2. Absorption time

The absorption time describes the time a diaper needs to distribute and absorb a defined quantity of a urine substitute applied on a defined area on the top sheet.

The baby diaper is mounted flat onto a foam support on the examination table in order to ensure a planar surface during measurement. The micturition point of the urine substitute is situated 2.5 cm ahead of the middle of the absorbent core.

The dosage unit consisting of a plate (10 x 30 cm, m = 500 g) and a cylinder with an integrated funnel having an addition nozzle connected to an opening in the plate having an inner diameter of 40 mm mounted thereon and a time measuring device is placed onto the diaper and weighted with 2 x 4 kg (ca. 27 g/cm²). Subsequently, an amount of liquid urine substitute is added to the baby diaper (total liquid amount: maxi size: 3 x 70 ml). The time required for complete absorption of the liquid is determined. After a defined waiting time of 5 min. the addition is repeated two times. The mean value of 5 individual measurements is given including standard deviation.

As can be taken from table 10, the results of the absorption time measurement show that a wet coating weight of 43 mg (V10) does not affect the absorption properties of the diaper. If applying higher wet coat weights, like in this case 250 mg (V8), the time to full absorption increases. However, the values still remain within the required time (< 200 s).

**Table 10**

| | First Addition | Second Addition | Third Addition |
|---|---|---|---|
| Reference (untreated diaper) | 34 | 79 | 114 |
| V8 | 46 | 108 | 171 |
| V10 | 35 | 76 | 113 |

### 4.3.3. pH value

The pH value on the diaper top sheet was determined by applying a defined quantity of a urine substitute and bringing a pH measuring stripe in contact with the non-woven surface after rinsing the diaper 1 to 3 times with the urine substitute.

As can be taken from table 11, the pH values are unobtrusive with respect to a wet coat weight of 43 mg (V8). At higher coat weights, the initially taken pH value increases, but is reduced after one rinsing to about 7. The pH on the diaper top sheet is a critical value because, values above 8 may increase the risk of the development of skin disorders.

**Table 11**

| | pH after first rinsing | pH after second rinsing | pH after third rinsing |
|---|---|---|---|
| Reference (untreated diaper) | 7 | 7 | 7 |
| V8 | 7 | 7 | 7 |
| V10 | 8 | 7 | 7 |

### 4.3.4. Microbiological Investigation

The contamination of the final, industrially coated diapers was examined following the European Pharmacopoeia (01/2011:50104; "Microbiological quality of non-sterile pharmaceutical preparations and substances for pharmaceutical use"), which requires
- a max. count of 200 (TVC/TAMC = Total Aerobe Microbial Count) and
- a max. count of 20 (TYMC = Total Yeast and Mould Count).

As can be taken from table 13, the testing results of the up to four weeks old diapers indicate no contamination as far as the method and the corresponding detection limits are set-up. The analysis of TAMC in V10 in Week 4 was declared as an error. The analysis of TAMC in V11 in Week 4 counted 5 TYMC, which is still within the requirements.

Aerobe bacteria *Pseudomonas aeruginosa*, *Staphylococcus aureus* and *Candida albicans* are completely absent.

**Table 13**

| **Sample** | **Weeks** | **TAMC** | **TYMC** | **Pseudomonas aeruginosa [g⁻¹]** | **Staphylococcus aureus [g⁻¹]** | **Candida albicans [g⁻¹]** |
|---|---|---|---|---|---|---|
| **Uncoated Reference [cfu/g]** | 1 | > 10⁴ | < 100ⁱⁱ | absent | absent | absent |
| | 2 | < 100 | 5ⁱⁱ | absent | absent | absent |
| | 3 | < 100 | 5ⁱⁱⁱ | absent | absent | absent |
| **V8** | 1 | < 100 | < 100ⁱⁱ | absent | absent | absent |
| | 2 | < 100 | < 100ⁱⁱ | absent | absent | absent |
| | 3 | < 100 | < 10ⁱⁱⁱ | absent | absent | absent |
| **V9** | 1 | < 100 | < 100ⁱⁱ | absent | absent | absent |
| | 2 | < 100 | < 100ⁱⁱ | absent | absent | absent |
| | 3 | < 100 | < 10ⁱⁱⁱ | absent | absent | absent |
| **V10** | 1 | < 100 | < 100ⁱⁱ | absent | absent | absent |
| | 2 | < 100 | < 100ⁱⁱ | absent | absent | absent |
| | 3 | < 100 | < 10ⁱⁱⁱ | absent | absent | absent |
| **V11** | 1 | < 100 | < 100ⁱⁱ | absent | absent | absent |
| | 2 | < 100 | < 100ⁱⁱ | absent | absent | absent |
| | 3 | < 100 | 5ⁱⁱⁱ | absent | absent | absent |

| | | | | | | |
|---|---|---|---|---|---|---|
| ⁱⁱ The detection limit was too high (100 cfu/g). Therefore, it was not possible to verify whether the acceptance criteria were met. ⁱⁱⁱ Additionally, the TYMC count was adjusted during the tests (in week 3) to verify the acceptance criteria of the EP: 1 g of the samples were mixed with 49 ml CASO-broth. 5 ml were plated by pour plate technique to lower the detection limit to 10 cfu/g. | | | | | | |

### 4.4. Drying Behaviour

In order to evaluate, whether the diapers coated with the inventive formulations according to the above described production process may also be dried during the coating process without affecting the top sheet substrate structure (PP fibres) due to melting or embrittlement, hence without deteriorating the performance of the diaper top sheet, the following trials were carried out.

For this purpose, not only spray coating, but also printing as alternative technology was tested to transfer the coating formulation to the nonwoven top sheet. This technology revealed to be a potential solution for an industrial upscale.

### 4.4.1. Materials and Methods

### Spray Coating Formulation

The spray coating formulation used for the coating of diapers and described in table 6 was used.

### Nonwoven material

The nonwoven top sheet substrate was a low bonded, soft PP (polypropylene) nonwoven top sheet typically used for standard baby diapers. Its average grammage is at 14 g/m², with a width of 175 mm.

### 4.4.2. Application of the coating formulation

The trial was realized on a lab printing machine (Testacolor 157, a laboratory printing press available from NSM Norbert Schläfli AG, Zofingen, Switzerland) using the above described spray coating equipment from FMP TECHNOLOGY GMBH. The spray equipment was assembled and installed on the printing machine.

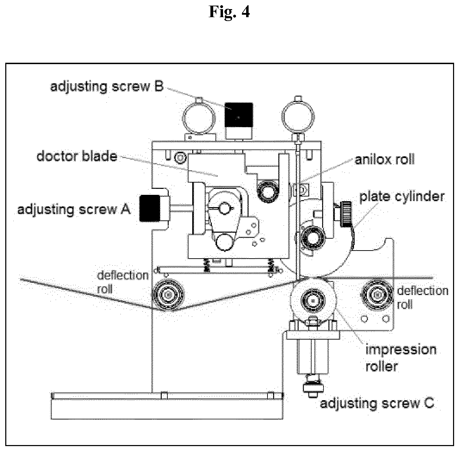

Additionally, one flexo printing unit was installed (cf. figure 4). Two dryers with a drying energy of up to 250 °C each were installed at the end of the line.
The following parameters were set to be met:

**Table 14:**

| | |
|---|---|
| Coat weight (wet) | 20 to 200 mg |
| Web speed | 100 m/min |
| Drying temperature | 100 to 250 °C |
| Coating length on top sheet | continuous |
| Coating width on top sheet | 45 ± 5 mm |

Two nozzle sizes were tested: 100 and 200 µm.

The pressure of the pressurized vessel was adjusted between 14 bar and 30 bar depending on the flowability of the operating nozzle. An observation of the abrasion tendency of coating material on machine parts was conducted on the transportation rolls, especially after the drying section.

In the subsequent printing application test, three different setups were used:
- Printing plate Flinn ART 2.54 mm, 360er Anilox roll
- Elastomer printing plate, 360er Anilox roll
- Elastomer printing plate, 120er Anilox roll

As a difference to the spray coating system which was adjusted to coat a width of 45 mm ± 5 mm, the printing system transfers the coating formulation on a width of 150 mm and in specific pattern, depending on the characteristic of the printing plate. The Elastomer printing plate allows full area coating onto the substrate.

Samples were taken by cutting 210 mm long pieces of spray coated or printed nonwoven from the reels.

### 4.4.3. Results and discussions

Tables 15 and 16 give overviews of the results of trials V12 to V16 using spray coating and V17 to V19 using printing, the used equipment and different settings (pressure vessel, dyers, temperature, etc.). For all trial points, the machine speed was set to 100 m/min.

Furthermore, the results of the determined residual moisture, the zinc content and the recalculated dry- and wet coat weights are displayed. The weight values are based on samples cut to a length of 210 mm.

### Residual moisture after coasting and drying

Different coat weights were determined using a four decimal digits lab balance (Mettler Toledo):
- Coat weight right after spraying/printing and drying
- Coat weight after storage under ambient conditions (air-dried, ca. 20 °C)
Based on these weights, the residual moisture after the coating and drying process was calculated

### Zinc content

The zinc content on the coated nonwoven was determined by extraction of solids on the coated top sheet in 25 ml of a 3% HNO₃ solution, shaking and letting stand over night. After filtration over a 0.2 µm regenerated cellulose syringe-filter, the solution was appropriately diluted before measuring. Zn-66 and Zn-68 were measured with an Inductively Coupled Plasma - Mass Spectrometer (ICP-MS) Elan DRCe from Perkin Elmer with an argon plasma flow of 15 l/min. Based on the zinc content, the dry- and wet coat weights were calculated

**Table 15**

| | **V12** | **V13** | **V14** | **V15** | **V16** |
|---|---|---|---|---|---|
| **Coating** | Spray | Spray | Spray | Spray | Spray |
| **Nozzle size [µm]** | 200 | 200 | 200 | 100 | 200 |
| **Pressure Vessel [bar]** | 14 | 14 | 12 | 30 | 14 |
| **Temp. Dryer 1 [°C]** | 160 | 250 | 220 | 160 | 160 |
| **Temp. Dryer 2 [°C]** | 130 | 230 | 200 | 140 | 140 |
| **Nonwoven Temp. Coated Area [°C]** | 35 | 40 - 45 | 35 - 40 | 35 | 35 |
| **Nonwoven Temp. Uncoated Area [°C]** | 45 - 50 | 60 - 65 | 45 - 50 | 45 | 45 |
| **Residual Moisture [%]** | 7.45 | 0.73 | 3.44 | 0 | 1.73 |
| **Zn content [mg]** | 0.49 | 0.64 | 0.68 | 0.19 | 0.37 |
| **Dry Coat Weight [mg]** | 38.04 | 49.27 | 52.62 | 14.31 | 28.38 |
| **Wet Coat Weight [mg]** | 120.76 | 156.41 | 167.03 | 45.42 | 90.11 |

To determine the temperature of the coated and uncoated nonwoven material after the drying section, the temperature measuring device Raynger ST2L from Raytek was used. The device uses non-contact, infrared measuring principle. The temperature is calculated based on the emitting radiation energy from the substrate. The measuring point was 30 cm after the last dryer. The distance from measuring device to substrate was ca. 20 cm .

As can be taken from table 15, the residual moisture content of V15 was best. However, the 100 µm nozzle tended to plug, such that a 200 µm nozzle was used, which produced nearly equally good results. V12, V13, V14 and V16 showed a residual moisture from 0.73 % to 7.45 % depending on the adjusted temperature of the dryers.

The preferred drying temperature was found to be 160 °C and lower. Higher drying temperatures of up to 250°C caused an increase of the temperature of the nonwoven itself and resulted in a fusing of the PP fibres.

This happened in the uncoated areas, where the temperature of the substrate reached 55 °C or higher. In coated areas, the fibres were unaffected because the substrate temperature at no time exceeded 45 °C.

The detected zinc content on the samples reflects dry coat weights from 28 mg to 52 mg or wet coat weights from 90 mg to 167 mg (using the 200 µm nozzle). With the 100 µm nozzle a low dry coat weight of 14 mg (or 45 mg wet coat weight) could be achieved.

**Table 16**

| | **V17** | **V18** | **V19** |
|---|---|---|---|
| **Coating** | Printing | Printing | Printing |
| **Printing Plates Anilox Roll Pick-up volume** | Flinn ART 2.54 mm 360er Anilox 4.9 m³/m² | Elastomer 360er Anilox 4.9 m³/m² | Elastomer 120er Anilox 15.2 m³/m² |
| **Temp. Dryer 1 [°C]** | 120 | 120 | 130 |
| **Temp. Dryer 2 [°C]** | 0 | 0 | 0 |
| **Nonwoven Temp. Coated Area [°C]** | 30 - 35 | 30 - 35 | 30 - 35 |
| **Nonwoven Temp. Uncoated Area [°C]** | 30 - 35 | 30 - 35 | 30 - 35 |
| **Residual Moisture [%]** | dry | dry | dry |
| **Zn content [mg]** | 0.03 | 0.03 | 0.1 |
| **Dry Coat Weight [mg]** | 2.15 | 2.08 | 7.69 |
| **Wet Coat Weight [mg]** | 6.84 | 6.59 | 24.42 |

In V 17 to V19 the coating was achieved by printing. Two different printing plates (Finn Art 2.54 mm and elastomer) where used combined with two different Anilox rolls having a difference in their pick-up volume (4.9 m³/m² or 15.2 m³/m²). By this approach, the amount of coating formulation transferred to the substrate was significantly reduced to 2 mg (dry), and 6 mg (wet), respectively, using the Anilox roll with a lower pick-up volume. A coat weight of almost 8 mg (dry) or 24 mg (wet) was achieved with an elastomer printing plate and a 120er Anilox roll. Due to the low coat weights spread on a bigger area (150 mm width), the nonwoven could be dried to 100 %. A drying temperature of 120 °C or 130 °C realized with one dryer was sufficient. The second dryer could be switched off.

As a consequence, low coat weights led to a lower amount of zinc on the substrate of 0.03 mg to 0.1 mg.

In contrast to the spray coating system, the coat weight would not be reduced by higher machine speeds in industrial scale, because the printing unit would operate at the same speed and transfer an unvarying amount of fluid. But, the coat weight, respectively, the transferred amount of fluid, can be adjusted depending on the design of the printing plate and the pick-up volume of the Anilox roll.

In V17 to V19, coating depositions within the machine or on machine parts could be reduced to a minimum, due to the fact that the nonwoven material could be effectively dried.

### Microscopic Analysis

To examine the influence of coating and drying on the nonwoven material, SEM (Scattering Electron Microscopy) pictures were taken with back-scattering electron detector and secondary electron detector. The investigation allows a visualization of the PP fibres and calcium carbonate structures down to a size of 2 microns.

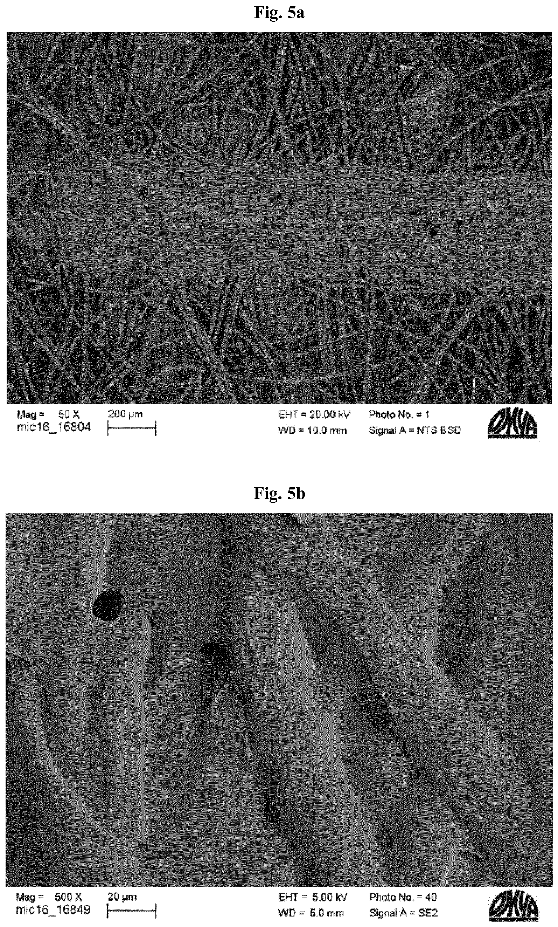

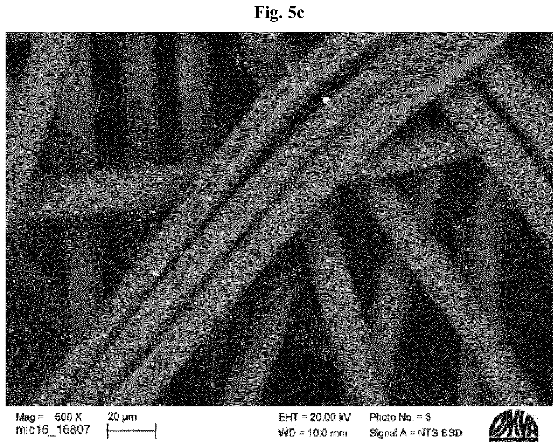

The SEM-analysis pictures of an uncoated reference nonwoven show a connection stripe, which is a results of the production process, in Figures 5a and 5b as well as a group of single fibres in Figure 5c under different magnification.

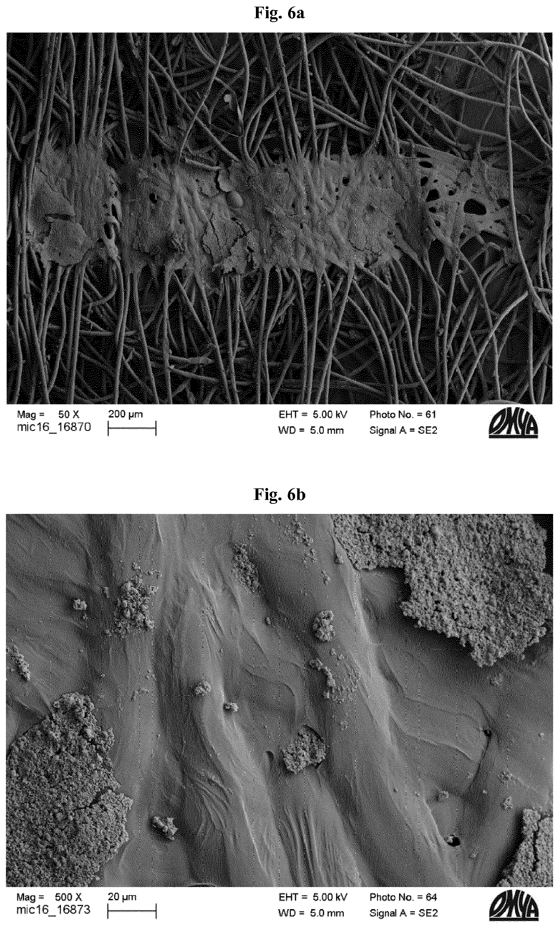

Figures 6a and 6b show the distribution of the spray coated zinc treated precipitated calcium carbonate solids on the nonwoven after coating according to V14.

The connection stripes visualized in Figures 6a and 6b, which are joining the fibres show accumulations of calcium carbonate. These kinds of nests are bonded to the substrate by the CMC binder which was incorporated in the formulation.

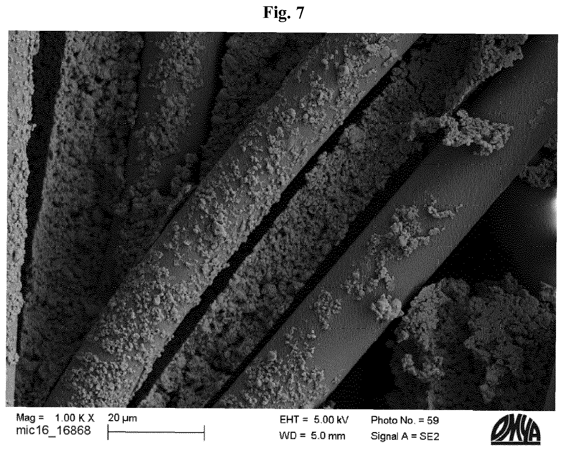

Besides the connection stripes, solids can be found widely distributed and bonded onto the PP fibres spray coated according to V13 (see Figure 7).

A destruction of the fibres in coated areas by melting or embrittlement due to the drying process could not be observed, but a fusing of the PP fibres in uncoated areas, where the temperature exceeded 55°C already became obvious by visual examination of the samples.

Figure 7 gives a good impression how the zinc containing calcium carbonate particles are spread on the PP fibres. The CMC as binding agent in the coating formulation offers good adhesion to the fibres and appears to be appropriate.

Figure 7 also gives an impression about the particle size which is about 2 µm in average.

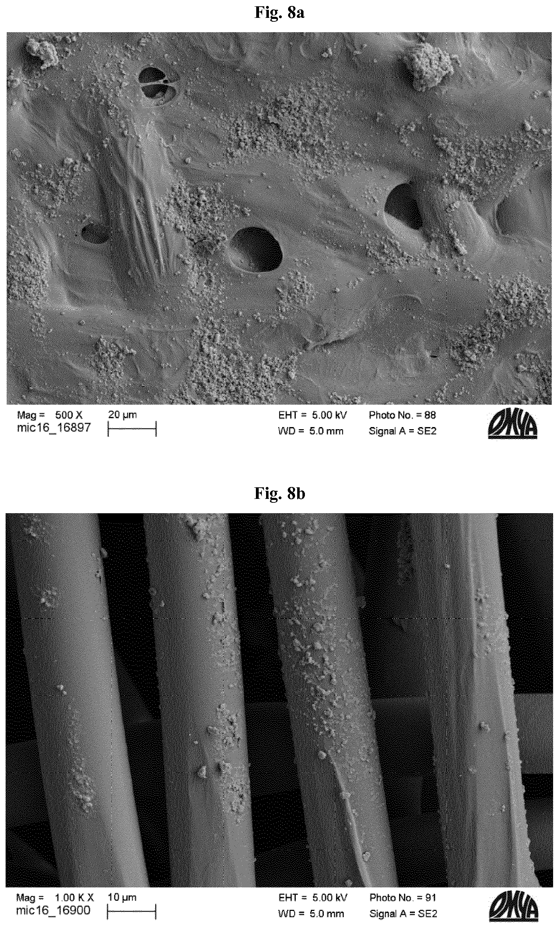

Compared to the spray coating application and as shown in the data table, lower amounts of solids were transferred by printing application which also becomes obvious in the SEM pictures of V17 in Figures 8a and 8b. However, a wide distribution is given as well.

### Tensile strength

As an indicative evaluation, the coated nonwoven samples were tested for their tensile strength using an ISO 527-3 tensile strength test method (typically applied on foils) on a Zwick Roell device with a 20 kN load cell. Stripes with a length of 150 mm and a width of 15 mm were punched out of the coated or uncoated area of the samples and placed into the sample holder. The test was conducted with a preload of 0.2 N and a test speed of 500 mm/min. The value are average values of 5 measurements, respectively.

The results of the tensile strength test are summarized in tables 17 and 18:

**Table 17**

| | **Ref.** | **V12** | **V13 center** | **V13 edge** | **V14** | **V15** | **V16** |
|---|---|---|---|---|---|---|---|
| **Dry Coat Weight [mg]** | - | 38.04 | 49.27 | - | 52.62 | 14.31 | 28.38 |
| **Force max [N]** | 7.94 | 9.96 | 10.12 | 15.06 | 10.71 | 9.04 | 8.43 |
| **Standard Deviation S [N]** | 0.62 | 0.82 | 1.17 | 0.48 | 1.19 | 0.17 | 0.65 |
| **Variance V [%]** | 7.79 | 8.2 | 11.52 | 3.17 | 11.1 | 1.83 | 7.69 |

**Table 18**

| | **Ref.** | **V17** | **V18** | **V19** |
|---|---|---|---|---|
| **Dry Coat Weight [mg]** | - | 2.15 | 2.08 | 7.69 |
| **Force max [N]** | 7.94 | 7.98 | 7.42 | 7.27 |
| **Standard Deviation S [N]** | 0.62 | 0.24 | 0.64 | 0.68 |
| **Variance V [%]** | 7.79 | 3.07 | 8.61 | 9.34 |

This test, even if it is not standardized for nonwoven testing, indicates an increase of the tensile strength in machine direction (MD) by coating the substrate with the inventive formulations. Increased coat weights also increase the maximum force (at least for trial point V12 and V13 Center). This effect can be ascribed to CMC binder in the formulation, increasing the bonding force of the material. Lower coat weights as achieved in V 17 to V19 do not have an influence on the tensile strength.

Testing the uncoated edges of samples of V 13, where the high drying temperature led to a melting of the fibres, reveals that this structural change also affects the tensile strength properties. The maximum force in this case increases. However, the negative effects like embrittlement or increased roughness would probably impact other diaper properties negatively, which is not accepted by the customer.

## Claims

1. Use of zinc treated precipitated calcium carbonate (PCC) in hygienic products,
**characterized in that** the zinc treated precipitated calcium carbonate is obtained by
- slaking calcium oxide with water to obtain a calcium hydroxide slurry,
- carbonating the calcium hydroxide slurry,
- adding a Zn²⁺ ion provider before and/or during the carbonation.

2. Use according to claim 1,
**characterized in that** the Zn²⁺ ion provider is selected from the group comprising zinc sulphate; zinc halides such as zinc chloride, zinc bromide, zinc iodide; zinc nitrate; zinc phosphates, e.g. Zn₃(POa)₂, ZnHPO₄; zinc carbonates, e.g. ZnCO₃, Zn(HCO₃)₂; zinc oxide; zinc hydroxide; hydrates and mixtures thereof.

3. Use according to any one of claims 1 or 2,
**characterized in that** the Zn²⁺ ion provider is added in an amount of from 0.1 to 30 wt%, preferably of from 2 to 25 wt%, more preferably of from 5 to 20 wt%, most preferably of from 10 to 15 wt%, e.g. 11.5 wt% based on the dry weight of calcium oxide.

4. Use according to any one of the preceding claims,
**characterized in that** the Zn²⁺ ion provider is added in combination with one or more Group I and/or Group II and/or Group III metal sulphates, which are preferably selected from the group comprising sodium sulphate, calcium sulphate, magnesium sulphate, aluminium sulphate; hydrates and mixtures thereof.

5. Use according to claim 4,
**characterized in that** the one or more Group I and/or Group II and/or Group III metal sulphates are added in an amount of from 0.1 to 30 wt%, preferably of from 1 to 25 wt%, more preferably of from 2 to 15 wt%, most preferably of from 4 to 8 wt%, e.g. 6 wt% based on the dry weight of calcium oxide.

6. Use according to any one of the preceding claims,
**characterized in that** the Zn²⁺ ion provider and/or the Group I and/or Group II and/or Group III metal sulphates, independently from each other are added before and/or during the carbonation.

7. Use according to any one of the preceding claims,
**characterized in that** that the calcium hydroxide slurry is screened before the addition of the Zn²⁺ ion provider and/or after the carbonation step.

8. Use according to any one of the preceding claims,
**characterized in that** the carbonation step is performed at a carbonation gas flow rate of below 30 litres per minute, preferably at a rate of from 1 to 30, more preferably of from 10 to 20, most preferably 20 litres per minute, and/or preferably at a temperature of from 10 to 70 °C, more preferably 15 to 50 °C, and most preferably 20 to 30 °C during precipitation.

9. Use according to any one of the preceding claims,
**characterized in that** after the carbonation the obtained precipitated calcium carbonate slurry is concentrated, optionally in the presence of cationic and/or anionic dispersants, preferably until a solids content of from 15 to 60 wt%, more preferably of from 20 to 55 wt%, especially preferably of from 25 to 50 wt%, most preferably of from 30 to 45 wt%, e.g. 35 to 40 wt% is obtained, or until dryness.

10. Use according to any one of the preceding claims,
**characterized in that** the zinc treated precipitated calcium carbonate is provided in the form of a formulation, especially an aqueous formulation, preferably selected from the group comprising coating formulations, spray coating formulations, lotions, printing ink formulations.

11. Use according to any one of the preceding claims,
**characterized in that** the zinc treated precipitated calcium carbonate is provided in the form of an aqueous formulation having a solid content of from 15 to 70 wt%, preferably 20 to 60 wt%, more preferably 30 to 40 wt%, e.g. 32 wt%, and/or a viscosity of from 50 to 3000 mPa·s, preferably from 100 to 2000 mPa·s, more preferably from 200 to 1000 mPa·s, especially preferably from 300 to 700 mPa·s, most preferably from 400 to 600 mPa·s, e.g. 460 mPa·s.

12. Use according to claims 10 to 11,
**characterized in that** the formulation comprises further components selected from the group comprising dispersing agents, binding agents, biocides, defoamers, oils, pigments, coalescing agents, wetting agents, neutralizing agents, emulsifiers, solvents, colorants, and mixtures thereof.

13. Use according to any one of the preceding claims,
**characterized in that** the zinc treated precipitated calcium carbonate in the form of a slurry, dry or in the form of a formulation is applied to hygienic products, especially ab/adsorbent products such as diapers, training panties, swim pants, feminine hygiene products such as pads, panty liners, sanitary napkins, incontinence products; deodorant formulations; nonwoven products such as wipes.

14. Use according to any one of the preceding claims,
**characterized in that** the zinc treated precipitated calcium carbonate in the form of a slurry, dry or in the form of a formulation is applied to an ab/adsorbent product comprising one or several layers selected from the group comprising a top sheet layer, one or more acquisition/distribution layer(s) (ADLs), a tissue wrap layer, an ab/adsorbent core and a back sheet layer.

15. Use according to claim 14,
**characterized in that** the zinc treated precipitated calcium carbonate in the form of a slurry, dry or in the form of a formulation is applied to one or several of the layers of the ab/adsorbent product, selected from the group comprising a top sheet layer, one or more acquisition/distribution layer(s) (ADLs), a tissue wrap layer, an ab/adsorbent core and a back sheet layer.

16. Use according to any one of the preceding claims,
**characterized in that** the zinc treated precipitated calcium carbonate is applied to provide a dry coating weight of 0.05 to 15 mg/cm², preferably 0.1 to 10 mg/cm², more preferably 0.2 to 5 mg/cm², most preferably 0.5 to 4 mg/cm², e.g. 1 to 2 mg/cm².

17. Hygienic product comprising zinc treated precipitated calcium carbonate as defined in any one of the preceding claims, wherein the hygienic products are selected from the group comprising ab/adsorbent products such as diapers, training panties, swim pants, feminine hygiene products such as pads, panty liners, sanitary napkins, incontinence products; deodorant formulations; nonwoven products such as wipes.

18. Hygienic product according to claim 17,
**characterized in that** the hygienic product is an ab/adsorbent product, preferably a diaper, comprising one or several layers selected from the group comprising a top sheet layer, one or more acquisition/distribution layer(s) (ADLs), a tissue wrap layer, an ab/adsorbent core and a back sheet layer, wherein zinc treated precipitated calcium carbonate as defined in any one of the preceding claims is applied to at least one of said layers.

19. Process for the preparation of a hygienic product according to any one of claims 17 to 18,
**characterized in that** the zinc treated precipitated calcium carbonate is applied to the hygienic product in the form of a slurry, dry, or in the form of a formulation.

20. Process according to claim 19,
**characterized in that** the zinc treated precipitated calcium carbonate is applied by spray coating or printing, e.g. flexographic printing.
